# EUROPEAN PATENT SPECIFICATION

(11) **EP 4 313 178 B1**
(45) Date of publication and mention of the grant of the patent: **12.02.2025**
(21) Application number: 22718785.3
(22) Date of filing: 01.04.2022
(51) Int. Cl.: A61L 2/18, A61L 2/20

(54) **DECONTAMINATION MACHINE AND CATALYSER DEVICE FOR THE TREATMENT OF CHEMICAL VAPOURS**
DEKONTAMINATIONSMASCHINE UND KATALYSATORVORRICHTUNG ZUR BEHANDLUNG CHEMISCHER DÄMPFE
MACHINE DE DÉCONTAMINATION ET DISPOSITIF CATALYSEUR POUR LE TRAITEMENT DES VAPEURS CHIMIQUES

(30) Priority: 01.04.2021 IT 202100008180
(43) Date of publication of application: 07.02.2024
(73) Proprietor: FEDEGARI AUTOCLAVI SPA, I-27010 Albuzzano (PV) (IT)
(72) Inventor: FEDEGARI, Giuseppe, 27100 Pavia (IT); FEDEGARI, Paolo, 7505 Celerina/Schlarigna (CH)
(74) Representative: Tarabbia, Luigi
(86) International application number: PCT/IB2022/053054
(87) International publication number: WO 2022/208456

(56) References cited:
- US-A- 5 711 705
- US-A- 5 711 705
- US-A1- 2009 047 173
- US-A1- 2009 047 173
- US-A1- 2013 336 844
- US-A1- 2013 336 844
- US-A1- 2020 360 551
- US-A1- 2020 360 551

## Description

### Technical field

The present invention relates to the technical sector of the production and packaging of sterile products in containers, for example powders or liquids. In particular, the present invention relates to a machine for the decontamination in particular of containers for pharmaceutical use.

### Prior art

The production and preparation of medical or chemical-pharmaceutical devices requires the application of specific decontamination protocols aimed at ensuring the safety and protecting the health of both patients and healthcare professionals who must use such devices.

The known decontamination protocols involve subjecting objects and substances to specific temperatures and pressures inside special decontamination machines.

For example, it is known from US5711705 an isolation work station comprising an enclosure, and an air circulation system and high efficiency air filter for generating a downwardly directed laminar air flow through the enclosure. Periodic sterilization of the enclosure may be accomplished by adding a sterilant, such as vaporized hydrogen peroxide, to the airstream, and the filter is impregnated with a catalyst for degrading the vaporized hydrogen peroxide during the purge cycle and wherein the airstream is circulated at a relatively low speed so as to increase the residence time in the filter.

It is also known from US2009/047173 a system for handling items including an enclosure, which is capable of being isolated from the surrounding environment and from a chamber of a decontamination system fluidly connected therewith. The enclosure includes an opening sized for receiving a container containing potentially contaminated items, such as incoming mail. Manipulators allow the mail to be sorted in the enclosure without risk of contaminating an operator or the surrounding environment. Objects to be decontaminated are conveyed between the enclosure and the chamber while being isolated from the surrounding environment. Vapor hydrogen peroxide or other decontaminant gas is used to decontaminate the enclosure, container, and any residual objects in the enclosure which are determined to be unsuited for ethylene oxide processing.

Furthermore, it is known from US2013/336844 an isolator including a contamination-target chamber including an inlet and outlet having intake and discharge filters, respectively; and a decontaminating-gas-supply unit to supply decontaminating gas into the chamber without flowing through the filters, the unit including: an atomizer including first and second ports and a nozzle; a first pipe having one and the other ends connected to a compressor and the first port, respectively; a second pipe having one end connected to the second port and the other end open; a reservoir; a pump to take in decontamination solution from the reservoir; and a third pipe, having one end connected to the pump, thorough which the solution flows, the atomizer configured to, when injecting from the nozzle intake air from the first port, suction the solution, via the second pipe, by negative pressure produced in the second port; and inject the solution in an atomized state, mixing it with air.

Last but not least, it is known from US2020/360551 a technique whereby hydrogen peroxide introduced into a working chamber containing a resin component is reduced to a low concentration in a shorter time than before: the result of this technique relies in a method of maintaining a sterile environment of a working chamber that contains a resin component, and includes a step (a) of introducing hydrogen peroxide into the working chamber, a step (b) of introducing air into the working chamber through a filter after completion of the step (a), a step (c) of generating ozone by irradiating the air with ultraviolet having a peak wavelength of from 160 nm to less than 200 nm upstream of the filter or inside the working chamber and introducing the ozone into the working chamber, and a step (d) of decomposing the ozone introduced into the working chamber into oxygen radicals.

During the treatment, it is known to introduce inside the protected environment chemical vapours comprising specific compounds particularly suitable for the abatement of the bacterial load, in particular one of the usable chemical compounds is hydrogen peroxide, whose disinfectant properties are also known and used in the home.

However, hydrogen peroxide, as well as other chemical agents usable in this context, in high concentrations or as a result of prolonged exposure is harmful to human health and it is therefore necessary to ensure that it is properly disposed of.

For this reason, there is a great interest in the sector in seeking to develop new solutions capable of ensuring the correct and efficient execution of decontamination processes, while avoiding possible risks to the environment and to human health.

### Object of the invention

In this context, the technical task underpinning the present invention is to propose a decontamination machine which obviates at least some of the drawbacks of the prior art cited above.

In particular, it is an object of the present invention to provide a decontamination machine capable of performing an efficient decontamination operation and at the same time disposing of the fluids used for such an operation in a safe, efficient and reliable manner.

The stated technical task and specified objects are substantially achieved by a decontamination machine, comprising the technical features disclosed in one or more of the appended claims.

According to the present invention, a machine is shown for the decontamination preferably of containers for pharmaceutical use, comprising a processing chamber, an inlet line, an outlet line and a catalyser device.

The processing chamber is configured to perform a decontamination process, of the chamber itself or its contents, and, as such, is hermetically sealable during the execution of a decontamination process.

The inlet line is configured to supply the processing chamber with a decontamination agent for the execution of the decontamination process.

The outlet line is configured to evacuate the decontamination agent from the processing chamber during a decontamination process.

The catalyser device is coupled to the outlet line and is configured to receive the decontamination agent and to promote a decomposition of at least one of its chemical components.

Advantageously, the machine described herein overcomes the drawbacks of the known art by allowing the chemical products used for the execution of the decontamination process to be abated and rendered harmless, thus safeguarding the health of the operators who use the machines and the air quality in the work environment.

At the same time, making the use of the chemicals present in the agents suitable for decontamination safer makes it possible to carry out more efficient and performing decontamination procedures.

The dependent claims, incorporated herein by reference, correspond to different embodiments of the invention.

### Brief description of the drawings

Further features and advantages of the present invention will become more apparent from the general and thus non-limiting description of a preferred, but not exclusive, embodiment of a decontamination machine, as illustrated in the accompanying drawings, in which:
- figure 1 schematically shows a machine adapted to perform a decontamination process;
- figure 2 shows a component of the machine of figure 1;
- figures 3A and 3B show respective details of the inner structure of the component of figure 2;
- figure 4 shows an embodiment of the component of figure 2 in a side view;
- figure 5 shows a plan view of figure 4;
- figure 6 shows a view from the outlet of the decontamination agent from the homogenisation module of figure 4.

### Detailed description of the preferred embodiments of the invention

In the accompanying drawings, reference number 1 generally indicates a decontamination machine, to which reference will be made in the following present description simply as machine 1.

The term decontamination means all those operations which can/must be carried out to obtain the abatement of the bacterial load present on the surfaces to be decontaminated.

By way of example, the containers to be decontaminated can be containers of the pharmaceutical sector, i.e., containers prepared for the storage and stocking of material and devices for medical use or drugs or other chemical compounds and components for health use or in any case in general containers for which it is necessary to abate the surface microbial load.

In particular, the machine 1 described herein comprises a processing chamber 2, an inlet line 3, an outlet line 4 and a catalyser device 5.

The processing chamber 2 is configured to contain a plurality of containers to be decontaminated, for example by means of suitable trays, racks, tube holders or other devices designed to support such containers.

The processing chamber 2 is also hermetically sealable with respect to an outside environment during the execution of a decontamination process.

In other words, the processing chamber 2 can be sealed with respect to an outside environment for the entire duration of the process with which the decontamination of the containers placed therein is performed or the decontamination of the chamber itself.

Advantageously, the processing chamber 2 can be further provided with a module for filling the containers and/or also be configured as a glove box to allow not only the decontamination of the containers, but also their filling and/or their handling to be carried out inside the processing chamber 2.

In particular, such filling can be performed in a chamber, previously decontaminated, automatically or manually; for example, it can occur by means of an injector which automatically introduces a chemical compound into the containers or by an operator who inserts the requested product into the container itself.

The chemical agent suitable for decontamination is introduced into the processing chamber 2 by means of the inlet line 3 which is configured to convey the decontamination agent by receiving it from one or more suitable supply tanks and/or lines.

The inlet line 3 can also be connected with different tanks and/or supply lines adapted to dispense separate compounds or chemical components which contribute to composing the decontamination agent.

The inlet line 3 can also have a plurality of injection nozzles of the decontamination agent in the processing chamber 2, so as to be able to feed the decontamination agent at several different points, allowing a better and more homogeneous distribution.

During the decontamination process, it is necessary to evacuate and subsequently dispose of the decontamination agent, so that an operator can open the processing chamber 2 at the end of the process without risking being exposed to the decontamination agent.

In particular, the removal of the decontamination agent from the processing chamber 2 is performed through the outlet line 4, which is specifically configured to evacuate the decontamination agent.

To avoid the dispersion in an outside environment of the decontamination agent, which is potentially harmful, along the outlet line 4, the catalyser device 5 is installed, which is configured to receive the decontamination agent and to promote a decomposition of at least one of its chemical components.

In other words, the catalyser device 5 promotes one or more chemical reactions which make the chemical compounds present in the decontamination agent harmless to human health by transforming them into non-hazardous compounds.

For example, in accordance with a preferred embodiment, the inlet line 3 is configured to supply a decontamination agent comprising hydrogen peroxide and the catalyser device 5 is configured to decompose the hydrogen peroxide.

Preferably, the action of the catalyser device 5 allows the following reaction to be catalysed:

2*H₂O₂ → 2*H₂O + O₂

Thereby, the hydrogen peroxide, potentially harmful to human health, is broken down into water and oxygen, which are harmless and can be released into an outside environment without posing any risk.

The present invention therefore involves the use of a catalyser device having one or more of the structural features described below to promote the establishment of chemical decomposition reactions in the decontamination agent leaving a machine 1.

Therefore, the installation of the catalyser device 5 on the machine 1 allows the decontamination operations to be performed safely, providing a safe and efficient decontamination agent disposal mechanism.

Structurally, the catalyser device 5 comprises a catalysis module 6 and a homogenisation module 7.

The catalysis module 6 in turn comprises a plurality of reciprocally abutting catalysis elements 6a arranged in series along a feed path of the decontamination agent inside the catalyser device 5.

Therefore, in use, during a decontamination procedure, the decontamination agent is evacuated from the processing chamber 2 through the outlet line 4.

Along its path inside the outlet line 4, the decontamination agent encounters the catalyser device 5 and passes through it in sequence through each of the catalysis elements 6a.

Thereby the decontamination agent is progressively decomposed, degrading its dangerous chemical components into compounds harmless to human health.

The specific number of catalysis elements 6a present inside the catalysis module 6 can be determined and selected as a function of the size of the machine 1, i.e., as a function of the concentration of decontamination agent and the air flow rate which is used by the machine 1.

In other words, on machines 1, of different dimensions and/or arranged to process different numbers of containers, different numbers of catalysis elements 6a could be installed.

The catalyser device 5, in general, and the catalysis module 6, in particular, are also configurable so as to be able to install on the machine 1 the most appropriate catalysis module 6 according to the size of the machine and the amount of containers which the machine must decontaminate during the execution of a certain decontamination process. The replaceability of the catalysis module 6 facilitates the execution of repair and maintenance operations on the machine 1.

In more detail, each catalysis element 6a comprises a frame, preferably a frame made of metal material (such as stainless steel) and a catalytic coating applied to such a frame and having thereon a surface density and a specific quantity which varies as a function of the required performance. In accordance with an aspect of the present invention, the frame of each catalysis element 6a has a honeycomb structure defining a plurality of linear conduits for the passage of the decontamination agent.

Such conduits extend parallel to the feed path
of the decontamination agent inside the catalysis module 6 and the conduits belonging to adjacent catalysis elements 6a can be reciprocally aligned so as to facilitate the flow of the decontamination agent therein.

Preferably, the catalytic coating has a chemical composition comprising platinum, which is particularly effective for promoting the aforementioned chemical reaction of hydrogen peroxide decomposition into water and oxygen.

The machine further comprises a recirculation circuit 8 which allows the decontamination agent to be brought back upstream of the catalyser device 5.

Thereby, if the action of the catalysis elements 6a has not been sufficient to completely eliminate the potentially harmful chemical compounds (or in any case to bring their concentration below a certain safety threshold value) it is possible to reintroduce the decontamination agent therein so as to subject it to a further decomposition process.

In particular, the recirculation line 8 returns the decontamination agent inside the processing chamber 2, from which it can then be transferred again through the outlet line 4 to the catalyser device 5.

Structurally, the recirculation circuit 8 has a first end arranged immediately downstream of the catalysis module 6 with respect to a feed direction of the decontamination agent in the outlet line 4.

In particular, the first end of the recirculation circuit 8 is coupled to the catalyser device 5 so as to be interposed between the catalysis module 6 and the homogenisation module 7.

The recirculation circuit 8 also has a second end coupled to an inlet line to the processing chamber 2 (i.e., opening into the processing chamber 2), preferably the second end of the recirculation circuit 8 is directly coupled at the processing chamber 2 to reintroduce the decontamination agent therein.

In use, the recirculation circuit 8 is selectively activatable to convey the decontamination agent upstream of the catalysis module 6.

In other words, the recirculation circuit 8 can be activated to withdraw the decontamination agent leaving the catalysis module 6 and return it upstream thereof, passing through the processing chamber 2, so as to allow it to pass through it again.

Downstream of the catalysis module 6 and upstream of the first end of the recirculation circuit 8 there is instead the homogenisation module 7 which is configured to homogenise a composition of the decontamination agent leaving the catalysis module 6 before introducing it into an outside environment.

In other words, when the recirculation channel 8 is closed, the homogenisation module 7 receives the decontamination agent from the catalysis module 6 and makes the concentration of the various chemical compounds comprising it homogeneous therein.

Thereby, the concentration of the chemical compounds reaches the same concentration in all the portions of the system and it is possible to precisely and accurately determine the actual concentration of the chemical compounds forming the decontamination agent without risking incorrect readings.

In detail, the homogenisation module 7 comprises an outer body 9, an intermediate body 10 and an inner body 11.

The outer body 9 has a substantially cylindrical shape extending between a first base wall 9a facing the catalysis module 6 and a second base wall 9b facing an outside environment.

The inner body 11, on the other hand, has a tubular shape extending at least partially inside the outer body 9 and having a first end 11a coupled to an outlet of the catalysis module 6, to receive the decontamination agent therefrom, and a second end 11b, directed towards the second base wall 9b.

The intermediate body 10 instead comprises a lateral wall 10a interposed between the inner body 11 and the outer body 9 and a transversal wall 10b interposed between the second end 11b and the second base wall 9b. The specific structural shape of the outer 9, intermediate 10 and inner 11 bodies defines an overall conveyance path for the decontamination agent having:
- a first portion "A" extending along the inner body 11;
- a second portion "B" extending between the inner body 11 and the intermediate body 10;
- a third portion "C" extending between the intermediate body 10 and the outer body 9.

To improve the structural stability of the homogenisation module 7 and facilitate the evacuation of the homogenised decontamination agent, the transversal wall 10b is constrained and spaced from the second base wall 9b and the latter has a plurality of homogeneously distributed holes 9c through which the decontamination agent is evacuated by the homogenisation module 7 and therefore by the catalyser device 6.

Depending on the type of chemical compounds present in the decontamination agent, the evacuation of the decontamination agent from the second base wall 9b can occur directly in the environment outside the machine 1, or in a further conduit which conveys the decontamination agent processed by the catalyser device 5 (and therefore rendered harmless to human health) to a suitable chimney arranged outside the work environment.

In order to validate the effectiveness of the catalysis process, a first sensor 12 is placed downstream of the homogenisation module 7, adapted to measure a concentration of at least one chemical component in the decontamination agent.

The machine 1 further comprises a second sensor adapted to measure the concentration of at least one chemical component in the decontamination agent positioned inside the processing chamber 2.

Optionally, the machine 1 can comprise a third sensor adapted to measure the concentration of at least one chemical component in the decontamination agent positioned downstream of the catalysis module 6 and upstream of the recirculation circuit 8 of the homogenisation module 7, i.e., upstream of the valves 13a and 13b.

By way of example, again in accordance with a preferred embodiment of the machine 1, the various sensors can be configured to measure a concentration of hydrogen peroxide in the decontamination agent.

Advantageously, the first sensor 12 makes it possible to measure the concentration of the chemical components present in the degraded decontamination agent leaving the catalyser device 5, in particular, leaving the homogenisation module 7, during the release into the environment outside the machine 1.

Furthermore, the first sensor 12 can generate an alert signal (e.g., an acoustic and/or optical signal) to alert an operator that the decontamination agent needs to be subjected to the decomposition process again.

Such a situation could arise if the catalyser device 5 loses efficiency over time

In other words, the decontamination agent is passed one or more times through the catalysis module 6, through the recirculation circuit 8, based on the concentration measurement of a certain chemical component inside the processing chamber.

In use, the machine 1 described herein makes it possible to perform a method for decontaminating surfaces and/or containers in a chamber 2 configured to be hermetically sealed so as to completely isolate its interior with respect to an outside environment (and therefore to the pollutants/pathogens potentially present therein).

Once the processing chamber 2 has been sealed, it is possible to perform one or more processes aimed at decontaminating the materials therein.

In particular, the method specifically involves a decontamination step in which a decontamination agent is injected inside the processing chamber 2, allowed to act and subsequently evacuated through an aeration step. At this point it is possible to open the processing chamber 2 and extract the material contained and decontaminated, or the processing chamber 2 can be further provided with a glove box to allow not only the decontamination, but also the handling of the components, to be carried out inside the processing chamber 2.

During the evacuation step (aeration) the decontamination agent is subjected to a decomposition step in which the chemical component is decomposed (for example the aforementioned hydrogen peroxide).

In greater detail, the decomposition step is performed by conveying the decontamination agent through the catalyser device 5, inside which the agent passes through a succession of catalysis elements 6a in series which promote a decomposition of the chemical compounds which compose it.

In accordance with a preferred embodiment, the decomposition step is performed by catalysing a decomposition reaction of the hydrogen peroxide, into water and oxygen.

At the end of the decomposition step, the now degraded decontamination agent is homogenised (through the homogeniser 7), and emitted in an environment outside the chamber 2. This occurs only in the decontamination step.

During the aeration step, the decontamination agent is degraded by its recirculating through the catalysis module 6; this occurs several times until the predefined threshold value is reached, in terms of concentration of the decontaminant, for the discharge of the material inside the chamber 2 or the start of a new handling activity thereof, by means of a glove box.

During the emission a concentration of at least one chemical component of the decontamination agent is measured and if the detected concentration is excessively high (e.g., above a predefined threshold value) the decontamination agent is recirculated to perform a further decomposition step.

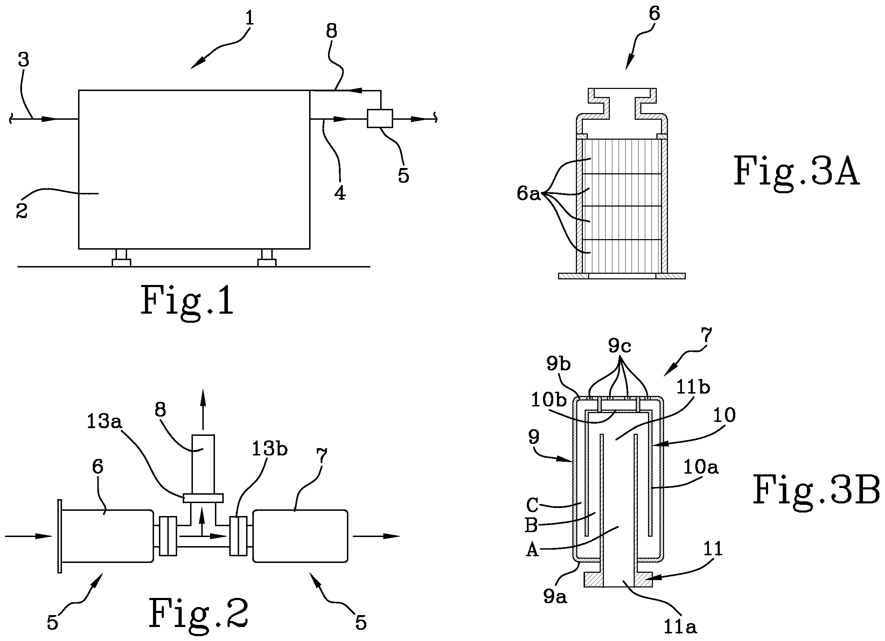

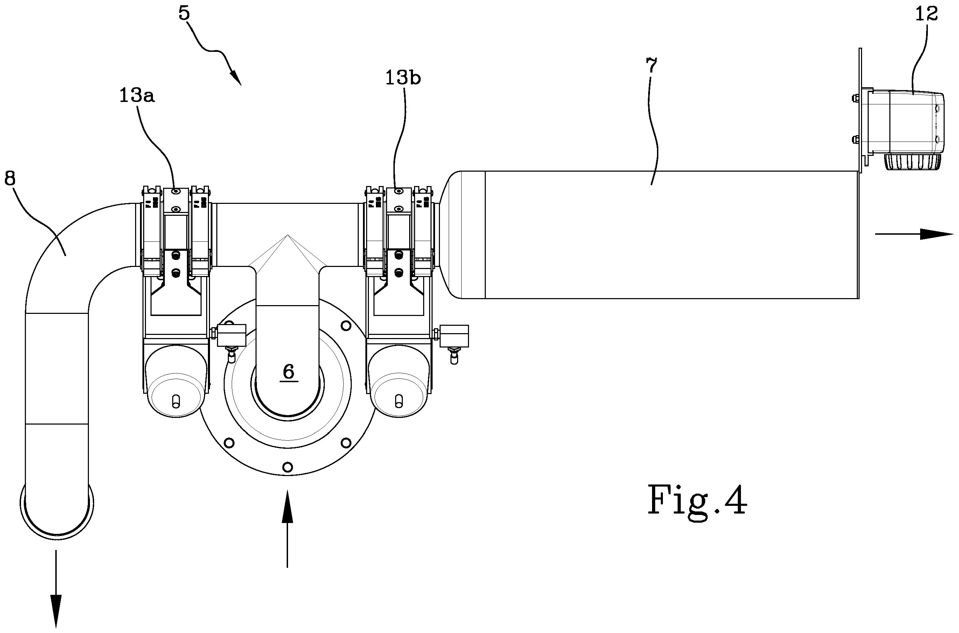

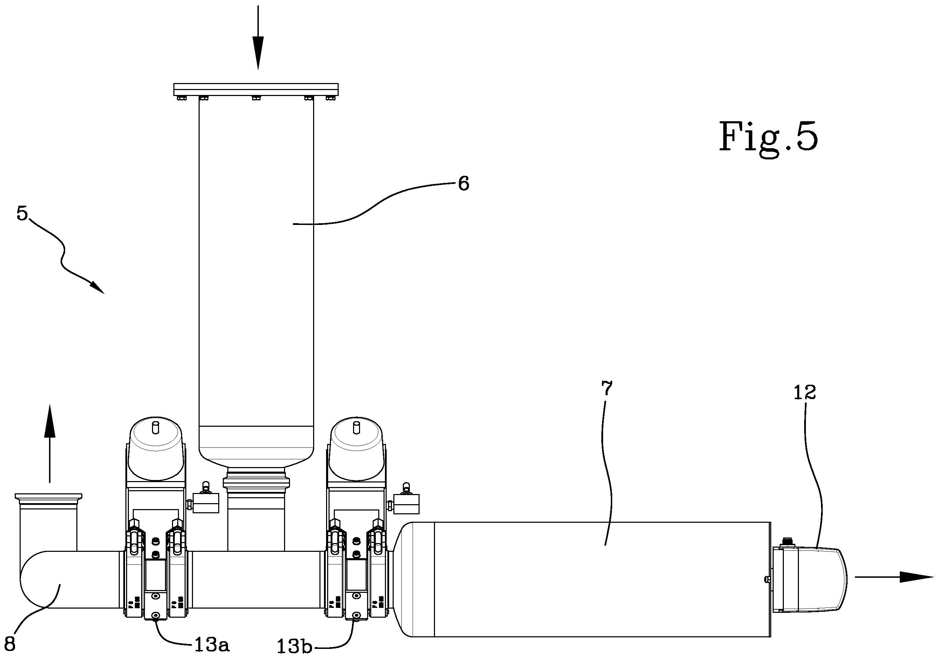

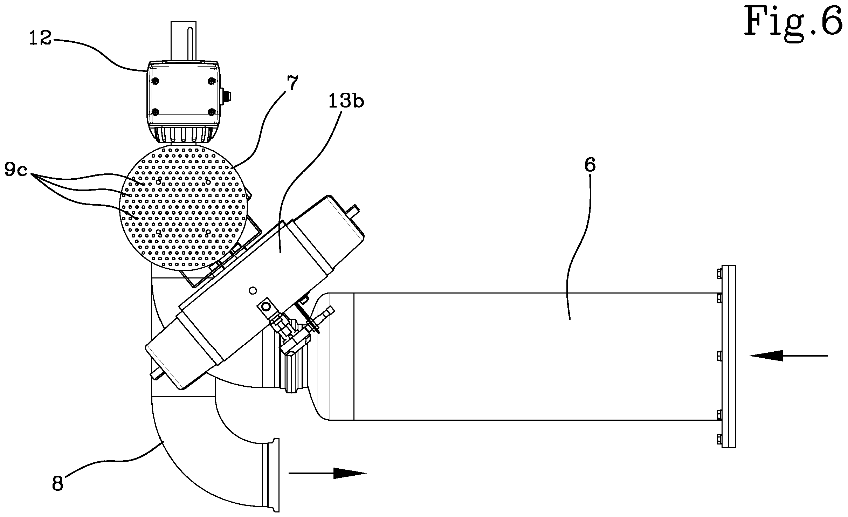

An embodiment of the catalyser of figure 2 is illustrated in figures 4, 5 and 6.

During the decontamination step, a decontamination agent is vaporised and injected inside the processing chamber 2 until a concentration, by non-limiting example, of 300 - 2000 ppm (preferably 800 - 1200) is reached, allowed to act for a variable duration of time (based on the selected concentration) and subsequently evacuated through the outlet line 4 and the catalyser 5.

The system is configured to selectively act on two motorised valves 13a, 13b, placed upstream of the homogenisation module 7 and upstream of the recirculation system 8. The two valves 13a, 13b work in an opposite manner, i.e., the opening of one requires the closure of the other.

In the decontamination step the decontaminating agent is degraded through its passage through the catalytic system 5. If the sensor 12 or the middle sensor detects (i.e., the third sensor) concentrations greater than 1ppm, the system enters emergency state, alerting a hazardous situation. In the decontamination step the first valve 13a is closed while the second valve 13b is open allowing the degraded decontaminating agent to pass through the homogenisation module 7.

Once the decontamination step inside the chamber 2 has been completed, the aeration step follows, in which a mixture of air and decontamination agent is circulated inside the catalysis module 6 one or more times, until the desired ppm is reached. In other words, during the aeration step, the decontamination agent begins to recirculate passing through the catalysis module 6 each time until the desired threshold level is reached, detected by a concentration sensor present inside the processing chamber 2 (not shown in the figures).

In the aeration step, the first valve 13a is open while the second valve 13b is closed.

Usually, once the sensors (second and third sensors) inside the machine have detected that the decontamination agent is suitably degraded, i.e., has reached a concentration < 1ppm, this is passed through the homogenisation module 7 and evacuated into the environment outside the machine 1.

Advantageously, the homogenisation module 7 supports the abatement of the concentration of decontaminant degraded by the catalysis module 6, by an extension of the exit time thereof from the machine 1, allowing it to further degrade spontaneously.

In place of the two motorised valves 13a, 13b described above, a three-way motorised valve can be used.

The presently claimed machine can be used for degrading a decontamination agent used during a decontamination step of inner surfaces of a decontamination machine 1 and/or containers, preferably containers for pharmaceutical use, comprising one or more passages through a catalysis module 6 and a single exit passage of the degraded decontamination agent through a homogenisation module 7.

Advantageously, the present invention achieves the proposed objects, overcoming the drawbacks complained of in the prior art by providing the user with a decontamination machine which can operate efficiently, decontaminating surfaces and/or materials which it processes without, however, at the same time generating risks to the health of the operators who use it or of those who are in transit in the work environment in which such a machine is installed.

## Claims

1. A machine for decontaminating surfaces and/or containers, preferably containers for pharmaceutical use, comprising:
- a processing chamber (2) configured to contain a plurality of containers to be decontaminated, said chamber (2) being hermetically sealable during the execution of a decontamination process;
- an inlet line (3) configured to feed to the processing chamber (2) a decontamination agent for the execution of said decontamination process;
- an outlet line (4) configured to evacuate the decontamination agent from the processing chamber (2) at the end of the decontamination process; and
- a catalyser device (5) coupled to the outlet line (4) and configured to receive the decontamination agent and to promote a decomposition of at least one chemical component of said decontamination agent,
**Characterized in that** it further comprises:
- a catalysis module (6) comprising a plurality of reciprocally abutting catalysis elements (6a) arranged in series along a feed path of the decontamination agent inside the catalysis module (6); and
- a homogenisation module (7) located downstream of the catalysis module (6) relative to said feed direction of the decontamination agent and configured to homogenise a composition of the decontamination agent leaving the catalysis module (6),
Wherein the homogenisation module (7) comprises:
- an outer body (9) having a substantially cylindrical shape, extending between a first base wall (9a) facing the catalysis module (6) and a second base wall (9b) facing an outside environment;
- an inner body (11) having a tubular shape, extending at least partially inside the outer body (9) and having a first end (11a) coupled to an outlet of the catalysis module (6) in order to receive the decontamination agent and a second end (11b) directed towards the second base wall (9b);
- an intermediate body (10) comprising a lateral wall (10a) interposed between the inner body (11) and the outer body (9) and a transversal wall (10b) interposed between the second end (11b) and the second base wall (9b), so as to define a conveyance path for the decontamination agent having:
- a first portion (A) extending in the inner body (11);
- a second portion (B) extending between the inner body (11) and the intermediate body (10);
- a third portion (C) extending between the intermediate body (10) and the outer body (9),
And wherein the transversal wall (10b) is constrained to and distanced from the second base wall (9b), said second base wall (9b) having a plurality of homogeneously distributed holes (9c).

2. The machine according to claim 1, wherein the inlet line (3) is configured to feed a decontamination agent comprising hydrogen peroxide and the catalyser device (5) is configured to decompose said hydrogen peroxide, preferably by catalysing the following reaction:
2 H₂O₂ → 2 H₂O + O₂

3. The machine according to claim 1, wherein each catalysis element (6a) comprises a frame, preferably a metal frame, and a catalytic coating applied to said frame, said catalytic coating preferably comprising platinum.

4. The machine according to claim 3, wherein each frame has a honeycomb structure defining a plurality of conduits for the passage of the decontamination agent, the conduits belonging to the frame of adjacent catalysis elements (6a) preferably being aligned, even more preferably coaxial.

5. The machine according to any one of the preceding claims, comprising a recirculation circuit (8) having a first end interposed between the catalysis module (6) and the homogenisation module (7) and a second end couplable to the outlet line (4) upstream of the catalysis module (6); said recirculation circuit (8) being selectively activatable so as to bring the decontamination agent back upstream of the catalysis module (6).

6. The machine according to any one of the preceding claims, comprising at least a first sensor adapted to measure a concentration of at least one chemical component in the decontamination agent at the homogenisation module (7), preferably downstream of the homogenisation module (7).

7. The machine according to any one of the preceding claims, comprising a second sensor adapted to measure a concentration of at least one chemical component in the decontamination agent at the catalysis module (6).

8. The machine according to claim 7, wherein said second sensor is positioned inside the processing chamber and/or in a position downstream of the catalysis module (6) and upstream of the recirculation circuit and upstream of the homogenisation module (70).

9. The machine according to claims 5, 7 or 8, wherein said at least one sensor is configured to generate a control signal adapted to activate the recirculation circuit (8) based on the concentration of the at least one chemical component in the decontamination agent.

10. The machine according to claim 6 or 7, wherein the at least one sensor comprises a sensor configured to measure a concentration of hydrogen peroxide in the decontamination agent.

## Patentansprüche

1. Maschine zur Dekontaminierung von Oberflächen und/oder Behältern, vorzugsweise von Behältern für pharmazeutische Zwecke, umfassend:
- eine Verarbeitungskammer (2), die so konfiguriert ist, dass sie eine Vielzahl von zu dekontaminierenden Behältern enthält, wobei die Kammer (2) während der Durchführung eines Dekontaminationsprozesses hermetisch verschließbar ist;
- eine Einlassleitung (3), die so konfiguriert ist, dass sie der Verarbeitungskammer (2) ein Dekontaminationsmittel für die Durchführung des Dekontaminationsprozesses zuführt;
- eine Auslassleitung (4), die so konfiguriert ist, dass sie das Dekontaminationsmittel am Ende des Dekontaminationsprozesses aus der Verarbeitungskammer (2) abführt; und
- eine Katalysatorvorrichtung (5), die mit der Auslassleitung (4) gekoppelt ist und so konfiguriert ist, dass sie das Dekontaminationsmittel aufnimmt und eine Zersetzung von mindestens einer chemischen Komponente des Dekontaminationsmittels fördert,
**Dadurch gekennzeichnet, dass** sie zudem Folgendes umfasst:
- ein Katalysemodul (6), das eine Vielzahl von wechselseitig aneinanderstoßenden Katalyseelementen (6a) umfasst, die in Reihe entlang eines Zufuhrpfades des Dekontaminationsmittels innerhalb des Katalysemoduls (6) angeordnet sind; und
- ein Homogenisierungsmodul (7), das stromabwärts des Katalysemoduls (6) in Bezug auf die Zufuhrrichtung des Dekontaminationsmittels angeordnet und so konfiguriert ist, dass es eine Zusammensetzung des Dekontaminationsmittels, das das Katalysemodul (6) verlässt, homogenisiert,
Wobei das Homogenisierungsmodul (7) umfasst:
- einen Außenkörper (9) mit einer im Wesentlichen zylindrischen Form, der sich zwischen einer ersten, dem Katalysemodul (6) zugewandten Basiswand (9a) und einer zweiten, der äußeren Umgebung zugewandten Basiswand (9b) erstreckt;
- einen rohrförmigen Innenkörper (11), der sich zumindest teilweise in das Innere des Außenkörpers (9) erstreckt und ein erstes Ende (11a) aufweist, das mit einem Auslass des Katalysemoduls (6) gekoppelt ist, um das Dekontaminationsmittel aufzunehmen, sowie ein zweites Ende (11b), das zur zweiten Basiswand (9b) gerichtet ist;
- einen Zwischenkörper (10), umfassend eine Seitenwand (10a), die zwischen dem Innenkörper (11) und dem Außenkörper (9) angeordnet ist, und einer Querwand (10b), die zwischen dem zweiten Ende (11b) und der zweiten Basiswand (9b) angeordnet ist, um einen Transportpfad für das Dekontaminationsmittel zu definieren, aufweisend:
- einen ersten Abschnitt (A), der sich in dem Innenkörper (11) erstreckt;
- einen zweiten Abschnitt (B), der sich zwischen dem Innenkörper (11) und dem Zwischenkörper (10) erstreckt;
- einen dritten Abschnitt (C), der sich zwischen dem Zwischenkörper (10) und dem Außenkörper (9) erstreckt,
Und wobei die Querwand (10b) an die zweite Basiswand (9b) gezwungen und von dieser beabstandet ist, wobei die zweite Basiswand (9b) eine Vielzahl von gleichmäßig verteilten Löchern (9c) aufweist.

2. Maschine nach Anspruch 1, wobei die Einlassleitung (3) so konfiguriert ist, dass sie ein Wasserstoffperoxid umfassendes Dekontaminationsmittel zuführt, und die Katalysatorvorrichtung (5) so konfiguriert ist, dass sie das Wasserstoffperoxid zersetzt, vorzugsweise durch Katalysieren der folgenden Reaktion:
2 H₂O₂ --> 2 H₂O + O₂

3. Maschine nach Anspruch 1, wobei jedes Katalyseelement (6a) einen Rahmen, vorzugsweise einen Metallrahmen, und eine auf den Rahmen aufgebrachte katalytische Beschichtung umfasst, wobei die katalytische Beschichtung vorzugsweise Platin umfasst.

4. Maschine nach Anspruch 3, wobei jeder Rahmen eine Wabenstruktur aufweist, die eine Vielzahl von Kanälen für den Durchgang des Dekontaminationsmittels definiert, wobei die zum Rahmen benachbarter Katalyseelemente (6a) gehörenden Kanäle vorzugsweise fluchtend, noch bevorzugter koaxial angeordnet sind.

5. Maschine nach einem der vorhergehenden Ansprüche, umfassend einen Rezirkulationskreislauf (8) mit einem ersten Ende, das zwischen dem Katalysemodul (6) und dem Homogenisierungsmodul (7) angeordnet ist, und einem zweiten Ende, das mit der Auslassleitung (4) stromaufwärts des Katalysemoduls (6) gekoppelt werden kann; wobei der Rezirkulationskreislauf (8) selektiv aktivierbar ist, um das Dekontaminationsmittel stromaufwärts des Katalysemoduls (6) zurückzubringen.

6. Maschine nach einem der vorhergehenden Ansprüche, umfassend mindestens einen ersten Sensor, der geeignet ist, eine Konzentration mindestens einer chemischen Komponente im Dekontaminationsmittel am Homogenisierungsmodul (7), vorzugsweise stromabwärts des Homogenisierungsmoduls (7), zu messen.

7. Maschine nach einem der vorhergehenden Ansprüche, umfassend einen zweiten Sensor, der geeignet ist, eine Konzentration mindestens einer chemischen Komponente im Dekontaminationsmittel am Katalysemodul (6) zu messen.

8. Maschine nach Anspruch 7, wobei der zweite Sensor innerhalb der Verarbeitungskammer und/oder in einer Position stromabwärts des Katalysemoduls (6) und stromaufwärts des Rezirkulationskreislaufs und stromaufwärts des Homogenisierungsmoduls (70) angeordnet ist.

9. Maschine nach Anspruch 5, 7 oder 8, wobei der mindestens eine Sensor so konfiguriert ist, dass er ein Steuersignal erzeugt, das geeignet ist, den Rezirkulationskreislauf (8) auf der Grundlage der Konzentration der mindestens einen chemischen Komponente im Dekontaminationsmittel zu aktivieren.

10. Maschine nach Anspruch 6 oder 7, wobei der mindestens eine Sensor einen Sensor umfasst, der so konfiguriert ist, dass er eine Konzentration von Wasserstoffperoxid im Dekontaminationsmittel misst.

## Revendications

1. Machine pour décontaminer des surfaces et/ou des récipients, de préférence des récipients à usage pharmaceutique, comprenant :
- une chambre de traitement (2) configurée pour contenir une pluralité de récipients à décontaminer, ladite chambre (2) pouvant être fermée hermétiquement pendant l'exécution d'un processus de décontamination ;
- une conduite d'entrée (3) configurée pour alimenter la chambre de traitement (2) en agent de décontamination pour l'exécution dudit processus de décontamination ;
- une conduite de sortie (4) configurée pour évacuer l'agent de décontamination de la chambre de traitement (2) à la fin du processus de décontamination ; et
- un dispositif catalyseur (5) couplé à la ligne de sortie (4) et configuré pour recevoir l'agent de décontamination et pour favoriser la décomposition d'au moins un composant chimique dudit agent de décontamination,
**caractérisée en ce qu'**elle comprend en outre :
- un module de catalyse (6) comprenant une pluralité d'éléments de catalyse (6a) se touchant réciproquement et disposés en série le long d'un chemin d'alimentation de l'agent de décontamination à l'intérieur du module de catalyse (6) ; et
- un module d'homogénéisation (7) situé en aval du module de catalyse (6) par rapport à ladite direction d'alimentation de l'agent de décontamination et configuré pour homogénéiser une composition de l'agent de décontamination sortant du module de catalyse (6),
le module d'homogénéisation (7) comprenant :
- un corps externe (9) de forme sensiblement cylindrique, s'étendant entre une première paroi de base (9a) orientée vers le module de catalyse (6) et une seconde paroi de base (9b) orientée vers un environnement extérieur ;
- un corps interne (11) de forme tubulaire, s'étendant au moins partiellement à l'intérieur du corps externe (9) et présentant une première extrémité (11a) couplée à une sortie du module de catalyse (6) afin de recevoir l'agent de décontamination et une seconde extrémité (11b) orientée vers la seconde paroi de base (9b) ;
- un corps intermédiaire (10) comprenant une paroi latérale (10a) interposée entre le corps interne (11) et le corps externe (9) et une paroi transversale (10b) interposée entre la seconde extrémité (11b) et la seconde paroi de base (9b), de manière à définir un chemin d'acheminement de l'agent de décontamination :
- une première partie (A) s'étendant dans le corps interne (11) ;
- une deuxième partie (B) s'étendant entre le corps interne (11) et le corps intermédiaire (10) ;
- une troisième partie (C) s'étendant entre le corps intermédiaire (10) et le corps externe (9),
et dans lequel la paroi transversale (10b) est contrainte et éloignée de la seconde paroi de base (9b), ladite seconde paroi de base (9b) ayant une pluralité de trous (9c) répartis de manière homogène.

2. Machine selon la revendication 1, dans laquelle la conduite d'entrée (3) est configurée pour alimenter un agent de décontamination comprenant du peroxyde d'hydrogène et le dispositif catalyseur (5) est configuré pour décomposer ledit peroxyde d'hydrogène, de préférence en catalysant la réaction suivante :
2 H₂O₂ --> 2 H₂O + O₂

3. Machine selon la revendication 1, dans laquelle chaque élément de catalyse (6a) comprend un cadre, de préférence un cadre métallique, et un revêtement catalytique appliqué sur ledit cadre, ledit revêtement catalytique comprenant de préférence du platine.

4. Machine selon la revendication 3, dans laquelle chaque cadre a une structure en nid d'abeille définissant une pluralité de conduits pour le passage de l'agent de décontamination, les conduits appartenant au cadre des éléments de catalyse adjacents (6a) étant de préférence alignés, encore plus de préférence coaxiaux.

5. Machine selon l'une quelconque des revendications précédentes, comprenant un circuit de recirculation (8) ayant une première extrémité interposée entre le module de catalyse (6) et le module d'homogénéisation (7) et une seconde extrémité pouvant être couplée à la conduite de sortie (4) en amont du module de catalyse (6) ; ledit circuit de recirculation (8) étant sélectivement activable de façon à ramener l'agent de décontamination en amont du module de catalyse (6).

6. Machine selon l'une quelconque des revendications précédentes, comprenant au moins un premier capteur adapté pour mesurer une concentration d'au moins un composant chimique dans l'agent de décontamination au niveau du module d'homogénéisation (7), de préférence en aval du module d'homogénéisation (7).

7. Machine selon l'une quelconque des revendications précédentes, comprenant un second capteur adapté pour mesurer une concentration d'au moins un composant chimique dans l'agent de décontamination au niveau du module de catalyse (6).

8. Machine selon la revendication 7, dans laquelle ledit second capteur est positionné à l'intérieur de la chambre de traitement et/ou dans une position en aval du module de catalyse (6) et en amont du circuit de recirculation et en amont du module d'homogénéisation (70).

9. Machine selon les revendications 5, 7 ou 8, dans laquelle ledit au moins un capteur est configuré pour générer un signal de commande adapté pour activer le circuit de recirculation (8) en fonction de la concentration d'au moins un composant chimique dans l'agent de décontamination.

10. Machine selon la revendication 6 ou 7, dans laquelle l'au moins un capteur comprend un capteur configuré pour mesurer une concentration de peroxyde d'hydrogène dans l'agent de décontamination.
